# EUROPEAN PATENT APPLICATION

(11) **EP 4 748 821 A1**
(43) Date of publication of application: **27.05.2026**
(21) Application number: 24215468.0
(22) Date of filing: 26.11.2024
(51) Int. Cl.: C07C 51/43, C07C 63/26

(54) **CRYSTALLIZATION METHOD FOR PURIFIED TEREPHTHALIC ACID**

(71) Applicant: DePoly SA, 1950 Sion (CH)
(72) Inventor: Sudan, Sylvain, 1814 La Tour-de-Peilz (CH)
(74) Representative: Prins Intellectual Property AG

(57) **Abstract**

The invention relates to a crystallization method for purified terephthalic acid (PTA) to obtain crystalline PTA with an average crystal size of approx. 30-150 microns, the method comprising in the following order the steps of: (a) providing in a reactor a solution of 2-13 wt% of purified metalated salt of terephthalic acid (M-TP) in water at a pH of 7; (b) heating the M-TP solution to a crystallization temperature of 40-90°C and stirring the solution; (c) adding H₂SO₄ dropwise at the crystallization temperature over a total addition time of at least 4 hours performing in the following order the steps of: (i) adding H₂SO₄ in the amount of 0.10-0.15 molar equivalent to adjust the pH of the M-TP solution to 4.7 - 5.5 to receive a first suspension of purified terephthalic acid (PTA); (ii) adding H₂SO₄ in the amount of 0.5-1.5 molar equivalent to the first suspension of PTA following a linear addition, a quadratic addition or a cubic addition, with a predefined addition rate, until the total addition time and 0.5-1.5 molar equivalent of H₂SO₄ is reached receiving a second suspension of PTA; (d) cooling down the second suspension of PTA to room temperature; (e) filtering the cooled second suspension of PTA to collect crystalline PTA; f) washing the filtered crystalline PTA with cold water; and (g) drying the washed crystalline PTA.

## Description

### Technical Field

The invention relates to crystallization method for purified terephthalic acid (PTA) to obtain crystalline PTA with an average crystal size of approx. 30 - 150 microns.

### Technical Background

Poly(ethylene terephthalate), widely known as PET, is a semicrystalline thermoplastic polyester that is used in a variety of industries in the form of fibres, sheets, films, and bottles. Its stability, high mechanical strength, high resistance to atmospheric and biological agents, and good aesthetic appearance has led to its prevalence in both the commercial and industrial sectors. While PET has become an inextricable part of our lives, environmental concerns have been raised about its pollution in our ocean and landfills. The environmental effects of PET production are not only limited to post-consumer PET contaminating the landfills It has also been reported that while other industrial sectors can lower their carbon footprint, the petrochemical industry which produces PET will ultimately increase their greenhouse gas emissions with increased PET production, thereby eroding climate benefits.

The most common industrial synthesis route of PET is through the polycondensation of ethylene glycol (EG) and dimethyl terephthalate (DMT) or purified terephthalic acid (PTA) using a continuous melt-phase polymerization process with temperatures of approximately 280°C. The base chemicals for this process (EG, DMT, and PTA) are typical bulk chemicals that the petrochemical industry obtains from catalytic reforming of petroleum naptha to paraxylene. Therefore, closing the recycling loop has a cascading effect from removing post-consumer waste from our environment to lowering greenhouse gas emission by increasing the supply of PTA within the market, and decreasing our reliance of PTA from the petroleum industry.

Following the consumption of PET, either commercially or industrially, users typically recycle the product(s) via four distinct methods, which are referred to as primary through quaternary recycling depending on the quality of the recycled product While primary recycling exclusively deal with industrial PET scrap and salvage, secondary recycling physically reprocesses consumer PET through grinding, washing, drying and reprocessing. However, the quality of PET obtained through secondary recycling is not virgin, and therefore much of it ends up being incinerated to recovery the energy content (quaternary recycling). Ultimately, tertiary recycling, or the depolymerization of PET to its starting monomers is the ideal method to close the recycling loop, as the monomers can be resold back to the chemical industry to form virgin PET, or other products.

An effective, inexpensive, robust and practical technology for degrading plastic waste, such as PET material, and simultaneously producing terephthalic acid (TPA), and/or ethylene glycol (EG) and/or other monomers that form plastic material is described in WO2020173961 by the same applicant.

WO2020173961 (incorporated herein by reference in its entirety) provides a method of alkaline hydrolysis of one or more plastic polymers into terephthalic acid (TPA) and/or ethylene glycol (EG) and/or other monomers that form the one or more plastic polymers. The method may output terephthalic acid that is contaminated or otherwise of a quality that requires further purification before further processing and use. Current methods of purifying TPA are energy intensive, and economically inefficient.

U.S. provisional application No. 63425771 by the same applicant (incorporated herein by reference in its entirety) describes a method of purifying terephthalic acid. The method includes contacting unpurified terephthalic acid with graphite, activated carbon, and molecular sieve to provide a reaction mixture, stirring the reaction mixture for a first certain period of time, filtering the reaction mixture, to provide a reaction mixture filtrate, providing graphite, activated carbon, and molecular sieve to the reaction mixture filtrate, stirring the reaction mixture filtrate for a second certain period of time, filtering the reaction mixture filtrate, to provide a reaction output solution and precipitating purified terephthalic acid (PTA) from the reaction output solution.

For PTA to be processed into polyethylene terephthalate (PET) and other polyester based polymers with the TPA monomer, crystalline PTA with a crystal size of 30 to 150 microns is required. This is to allow efficient handling and flowability, and also to allow the optimal ratio of the PTA and other monomer (ethylene glycol in the case of PET) to form a paste suitable for the polymerisation process.

Common methods of purifying TPA to obtain purified terephthalic acid (PTA) typically include recrystallization steps, which require high temperatures (>100 °C) and pressures (>1 atm) to get the crystal of PTA to the right size (avg. 100 microns) for a later polycondensation process. The crystallization process is energy intensive and requires the use of harmful solvents, such as dimethylformamide. Such solvents may risk operator safety and require expensive, time consuming and complicated procedures to handle and manage.

Industrial crystallization is an effective means of purification for many organic and inorganic compounds. Under crystallization, there are two types of nucleation, primary nucleation and secondary nucleation. Primary nucleation is rapid and often results in small, irregularly shaped particles, meaning it is not often used in industry because of the risk of nucleation on foreign particles, thus occluding impurities into the crystal. Secondary nucleation occurs when the level of supersaturation is high enough for crystals to form in the presence of other crystals of the same material. Secondary nucleation is generally used in Industry and is also found in the crude terephthalic acid (CTA) purification process.

Industrial PTA is thus produced in a two-step process: (1) The first stage of the process includes *p*-xylene oxidation to produce CTA in a powder form. The impurity 4-CBA is a byproduct of this process. CTA powder is mixed with de-ionised water to form a slurry that is passed through the next stage i.e. purification. (2) The CTA purification process is carried out at high temperature and high pressure. Powdered CTA (29 wt%) is mixed with de-ionised water (71 wt%) to form a slurry. The slurry is heated to 287°C and pumped to a hydrogenation reactor operating at 79 bar. The slurry reacts with Nitrogen and hydrogen in the presence of a carbon-coated palladium catalyst to convert 4-CBA to p-toluic acid. The slurry, with acceptable levels of 4-CBA (<25ppm) is passed through multi-stage flash crystallization where the primary contaminants *p*-toluic Acid and benzoic acid are removed. [1], [2]

The retention time in each crystallizer is reported as 20 - 50 minutes in order to target a crystal size of 118 microns [1]. The 5-stage crystallization step is thus estimated to take approximately 1.5 - 4 hours.

In newer iterations of the crystallization process, more solvents have been introduced to decrease the time required to reach the required purity. These solvents typically used are acetic acid, DMSO, DMF, DMA, alcohols, and in some iterations involve the use of a catalysts such as Mn, Br, Co, Zn. However, in all cases, there is an energy penalty associated with the use of high temperatures and pressures in the conversion of CTA to PTA.

An important factor in the production of purified terephthalic acid is the formation of crystals of a size and shape that allow good handling, washing and filtration properties in the PTA production process, as well as easier handling and better processability in the polyester process.

### References:

[1] Online dual updating with recursive PLS model and its application predicting crystal size of PTA process (Mu et al, 2005, China). DOI:10.1016/j.jprocont.2005.11.004.
[2] Control structure design of a crude terephthalic acid hydropurification process with catalyst deactivation (Li et al, 2016, China). DOI:10.1016/j.compchemeng.2016.01.017

### Summary of the Invention

It is an objective of the invention to reduce energy consumption and costs in the preparation of purified terephthalic acid (PTA) obtained by chemical recycling of PET, thus lowering the energy consumption and costs of the overall recycling process of PET. In addition, it is an objective of the invention to improve the quality and processability of the purified terephthalic acid (PTA) which then can be used as monomers for the synthesis of PET.

At least one of the objectives of the present invention is achieved by a crystallization method according to claim 1. The crystallization method for purified terephthalic acid (PTA) to obtain crystalline PTA with an average crystal size of approx. 30-150 microns (µm) comprises in the following order the steps of: (a) providing in a reactor a solution of 2-13 wt% of purified metalated salt of terephthalic acid (M-TP) in water at a pH of 7; (b) heating the M-TP solution to a crystallization temperature of 40-90°C and stirring the solution; (c) adding H₂SO₄ dropwise at the crystallization temperature over a total addition time of at least 4 hours, preferably 4 to 10 hours, under continuous agitation performing in the following order the steps of: (i) adding H₂SO₄ in the amount of 0.10-0.15 molar equivalent to adjust the pH of the M-TP solution to 4.7 - 5.5, which is called nucleation point, until the formation of a suspension of PTA to receive a first suspension of purified terephthalic acid (PTA); and (ii) adding H₂SO₄ in the amount of 0.5-1.5 molar equivalent to the first suspension of PTA following a linear addition, a quadratic addition or a cubic addition, with a predefined addition rate, until the total addition time and 0.5-1.5 molar equivalent of H₂SO₄ is reached receiving a second suspension of PTA, wherein the predefined addition rate of the linear addition is defined by V(t) = Vf * (t / tf), the predefined addition rate of the quadratic addition is defined by V(t) = Vf * (t / tf)^2, and the predefined addition rate of the cubic addition is defined by V(t) = Vf * (t / tf)^3, where V(t) is the added acid volume at a given time, Vf is the total added acid volume, t is the time and tf is the total addition time; (d) cooling down the second suspension of PTA to room temperature; (e) filtering the cooled second suspension of PTA to collect crystalline PTA; (f) washing the filtered crystalline PTA with cold water; and (g) drying the washed crystalline PTA.

Previous known processes used acetic acid and/or heat/cool cycles to obtain PTA of the required quality to be further used for the production of PET. However, the use of acetic acid and temperature swings over an extended time period is costly and energy intensive. In addition, the resulting PTA could be improved to better fit the current industrial standards. The method of the present invention overcomes the limitations of the prior art and provides a cheaper and energy saving method for the crystallisation of PTA of high quality and processability, in combining the steps of predefined addition of sulfuric acid and seeding.

Switching from acetic to sulfuric acid brings an important cost reduction. Not only the market price for sulfuric acid is approximately 10x lower, but the use of a strong sulfuric acid (as opposed to the weak acetic acid) allows to use less molar equivalents (smaller volume of acid) for the precipitation. A key challenge that needed to be addressed was the formation of PTA particles with the desired size and quality. Classical, uncontrolled addition of a strong acid to a solution of disodium terephthalate (Na-TP) usually leads to the formation of very small particles that tend to aggregate. This makes the resulting PTA unsuitable for the following polymerization to recycled PET (rPET): low crystal quality leading to poor processability. The inventive method involves the slow addition of sulfuric acid following a specific profile. The controlled addition results in the formation of PTA particles that display a comparable quality to the ones that fit the industrial standard. The duration of the precipitation could also be reduced. Thus, the quality of the crystalline PTA can be improved by combining the slow addition with a seeding step leading to an unprecedented quality of the crystalline PTA, also called recycled PTA (rPTA) for the polymerization of PET.

In some embodiments the metalated salt of terephthalic acid (M-TP) may be obtained from depolymerization of waste PET via alkaline hydrolysis, preferably via the method as described in WO2020173961 and incorporated herein by reference in its entirety. In some embodiments the M-TP, which may be obtained from depolymerization of waste PET, may be further purified using graphite, activated carbon and molecular sieve to remove organic and inorganic contaminants. The purification may be performed with the method as described in U.S. provisional application No. 63425771 by the same applicant and incorporated herein by reference in its entirety. The output is typically a metalated salt of terephthalic acid (M-TP, with "M" denoting a metal such as Na or K that may originate from a hydrolysis process) dissolved in water, in solution at 2 to 13% by weight. The output may then be subjected to the crystallization method.

In some embodiments the metalated salt of terephthalic acid (M-TP) may be either disodium terephthalate (Na-TP) or potassium terephthalate (K-TP).

The M-TP solution is heated to a crystallization temperature of 40-90°C by stirring the solution. In a preferred embodiment the M-TP solution is heated to a crystallization temperature of 80-90 °C. The actual temperature in the reactor may defer by a few degrees from the set temperature.

In a preferred embodiment the method is performed under normal atmospheric pressure. In some embodiments the method may be performed at ambient pressure.

An important feature of the inventive crystallization method is the multi-step addition of the sulphuric acid over a total addition time of at least 4 hours, preferably 4 to 10 hours, more preferably 4 to 6 hours, under continuous agitation and at the crystallization temperature. In a first step, in step (c)(i), the sulphuric acid (H₂SO₄) is added, preferably in a fast addition, preferably with a linear rate, in the amount of 0.10-0.15 molar equivalent to adjust the pH of the M-TP solution to 4.7 - 5.5, preferably to 5.1 - 5.3, receiving a first suspension of PTA. This first addition is necessary to adjust the pH to 4.7 - 5.5, preferably to 5.1 - 5.3. After this point, the pH is low enough that the precipitated PTA cannot fully redissolve. This can be described as nucleation point. Fast addition herein means an addition time of preferably at the most twenty minutes, preferably between ten and twenty minutes. Thus, in a preferred embodiment, the sulphuric acid is added in the first step in a fast addition over a first addition time of twenty minutes or less with a linear rate to receive the nucleation point faster and to maximize the duration of the crystal growth period. The nucleation point refers to the initial stage of crystal formation, where molecules begin to arrange themselves into a structured lattice to form a crystal. This process occurs when the conditions (such as pH, temperature, pressure, and concentration) are right for the substance to transition from a disordered, liquid, or gas phase into a solid phase.

In a second step, in step (c)(ii), additional H₂SO₄ is added in the amount of 0.5-1.5 molar equivalent to the first suspension of PTA following a linear addition, a quadratic addition or a cubic addition, with a predefined addition rate, until the total addition time and 0.5-1.5 molar equivalent of H₂SO₄ is reached receiving a second suspension of PTA, wherein the predefined addition rate of the linear addition is defined by V(t) = Vf * (t / tf), the predefined addition rate of the quadratic addition is defined by V(t) = Vf * (t / tf)^2, and the predefined addition rate of the cubic addition is defined by V(t) = Vf * (t / tf)^3, where V(t) is the added acid volume at a given time, Vf is the total added acid volume, t is the time and tf is the total addition time. The addition in the second step (c)(ii) is a slow addition compared to the fast addition in the first step (c)(i). Slow addition herein means an addition time of preferably at least three hours, preferably 4 - 10 hours. Thus, in a preferred embodiment, the sulphuric acid added in the second step is added in slow addition over a second addition time of at least three hours, preferably 4 - 10 hours. The total addition time of the multi-step addition of the sulphuric acid is the sum of the first fast addition time plus the second slow addition time and is at least 4 hours, preferably 4 to 10 hours.

In a preferred embodiment, in the second step, the H₂SO₄ is added in the amount of 0.7 - 1.1 molar equivalent to the first suspension of PTA, more preferably in the amount of 0.9 - 1.0 molar equivalent.

In a preferred embodiment, the addition of H₂SO₄ in step (c)(ii) follows a cubic addition with a predefined addition rate defined by V(t) = Vf * (t / tf)^3, and V(t) is the added acid volume at a given time, Vf is the total added acid volume, t is the time and tf is the total addition time. The advantage of the cubic addition is that it maximizes crystal growth. The slow addition at the beginning allows to create less nucleation points compared to a faster addition rate which results in fewer but larger particles.

Preferably, the crystallization method of the present invention comprises an additional step (c)(ia) after step (c)(i) between the steps (c)(i) and (c)(ii). In the additional step (c)(ia), the first suspension of PTA after step (c)(i) is seeded with crystalline PTA seeds at the pH of 4.7-5.5. The crystalline PTA seed is a small, pre-formed piece of crystal that acts as a template or nucleus for further crystal growth. It provides a stable surface for atoms, molecules, or ions to organize themselves into a crystal lattice. The seeding with crystalline PTA seeds accelerates the crystallization process and results in the formation of a larger crystal which perfectly fit the industrial standard.

Thus, in a preferred embodiment, the crystallization method for purified terephthalic acid (PTA) to obtain crystalline PTA with an average crystal size of approx. 30-150 microns (µm) comprises in the following order the steps of: (a) providing in a reactor a solution of 2-13 wt% of purified metalated salt of terephthalic acid (M-TP) in water at a pH of 7; (b) heating the M-TP solution to a crystallization temperature of 40-90°C and stirring the solution; (c) adding H₂SO₄ dropwise at the crystallization temperature over total addition time of at least 4 hours, preferably 4 to 10 hours, under continuous agitation performing the steps of: (i) adding H₂SO₄ in the amount of 0.10-0.15 molar equivalent to adjust the pH of the M-TP solution to 4.7 - 5.5, which is called nucleation point, until the formation of a suspension of PTA to receive a first suspension of purified terephthalic acid (PTA); (ia) seeding the first suspension of PTA with crystalline PTA seeds; and (ii) adding H₂SO₄ in the amount of 0.5-1.5 molar equivalent to the first suspension of PTA following a linear addition, a quadratic addition or a cubic addition, with a predefined addition rate, until the total addition time and 0.5-1.5 molar equivalent of H₂SO₄ is reached receiving a second suspension of PTA, wherein the predefined addition rate of the linear addition is defined by V(t) = Vf * (t / tf), the predefined addition rate of the quadratic addition is defined by V(t) = Vf * (t / tf)^2, and the predefined addition rate of the cubic addition is defined by V(t) = Vf * (t / tf)^3, where V(t) is the added acid volume at a given time, Vf is the total added acid volume, t is the time and tf is the total addition time; (d) cooling down the second suspension of PTA to room temperature; (e) filtering the cooled second suspension of PTA to collect crystalline PTA; (f) washing the filtered crystalline PTA with cold water; and (g) drying the washed crystalline PTA.

In a preferred embodiment, the seeds are added as a solid. Preferably, the crystalline PTA seeds have an average crystal size which is smaller than the average size of the final crystalline PTA produced by the crystallization method of the invention. Preferably, the average size of the crystalline PTA seeds is approx. a third of the average size of the final crystalline PTA, preferably approx. 10-50 microns, while the average size of the final crystalline PTA is approx. 30-150 microns. The measured average particle size is referring to the median value for longest distance in the particle. In a preferred embodiment, the amount of crystalline PTA seeds added in step (c)(ia) to the first suspension of PTA is 1-10 wt% of the total weight of PTA in the M-TP solution of step (a). In one embodiment the crystalline PTA seeds are produced by a method of claim 1.

In one embodiment, in a first round, the crystallization method of the invention without the seeding step (c)(ia) is used to produce crystalline PTA seeds for seeding. In a second round, the crystallization method of the invention is performed adding crystalline PTA seeds in step (c)(ia) after step (c)(i), and the crystalline PTA seeds produced in the first round of the method of the invention are added in step (c)(ia) to seed the first suspension of PTA.

In another embodiment, any crystalline PTA seeds can be used in step (c)(ia) to seed the first suspension of PTA.

The crystallization method of the present invention with the multi-step addition of the sulphuric acid results in reduced acid consumption, reduced acid price, reduced time, improved quality of the crystalline PTA and reduced waste production (wash water) compared to the methods known in the art. The best results are achieved with the cubic addition of the sulfuric acid in step (c)(ii) and with seeding the first suspension of PTA after step (c)(i) with crystalline PTA seeds. The crystallization method including the seeding step improves the quality of PTA and increases the size of crystalline PTA which results in a crystalline PTA that is easier to process when later used for PET polymerisation.

After the multi-step addition of sulfuric acid, the second suspension of PTA is cooled down to room temperature, filtered, and the crystalline PTA is collected. In a preferred embodiment, the filtered crystalline PTA is washed with cold water, preferably with deionised water until the conductivity of the filtrate reaches a value below 50 µS/cm, and then dried, e.g. overnight in the oven at about 100 °C. In some embodiments the cold water of step f) may have a temperature of approx. 5 to 25°C.

Further embodiments of the invention are set forth in the dependent claims.

### Brief Explanation of the Figures

The invention is described in greater detail below with reference to embodiments that are illustrated in the figures. The figures show:
- Fig. 1: a description of the aspect ratio of a crystal;
- Fig. 2a: an addition profile of sulphuric acid equivalent over time (h) without initial fast addition;
- Fig. 2b: an addition profile of sulphuric acid equivalent over time (h) with initial fast addition;
- Fig. 3: the sulfuric acid addition profile of Fig. 2b with the dots connected;
- Fig. 4a: a microscopy image of PTA crystals produced without seeding; and
- Fig. 4b: a microscopy image of PTA crystals produced with seeding.

### Embodiments of the Invention

The unpurified terephthalic acid may be sourced from the output of a polymer recycling process, such as the room temperature alkaline polymer hydrolysis process described in PCT Application Publication No. WO2020173961A1.

Such unpurified terephthalic acid may comprise contaminants including, solid pigments such as carbon black, isostructural monomers such as phthalic acid, isophthalic acid, as well as molecules that can be present as contaminants in PET such as benzoic acid, p-toluic acid, bisphenol A, 4-carboxybenzaldehyde, metal ions and/or other contaminants originating from the waste PET. To remove the impurities, the unpurified terephthalic acid may be subject of a purification process described in U.S. provisional application No. 63425771.

The purified terephthalic acid resulting from the purification process is in the form of M-TPA (with "M" denoting a metal such as Na⁺ or K⁺ that may originate from a hydrolysis process) dissolved in water, in solution at ~ 2% to 13% by weight.

### Quality assessment

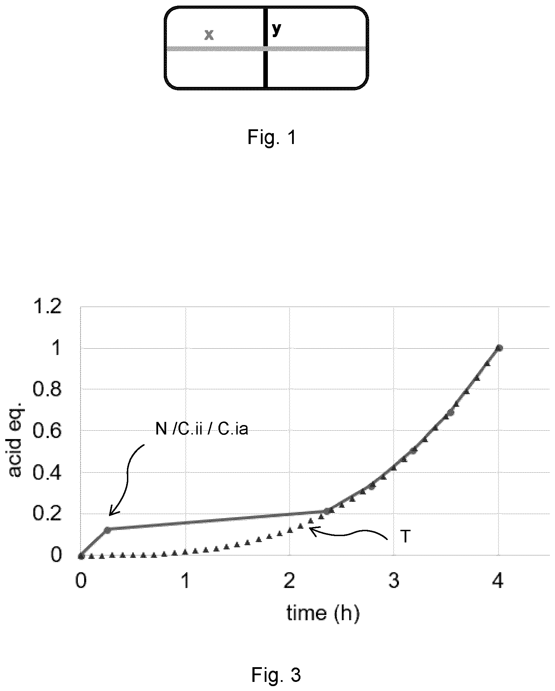

An important step in the synthesis of PET is the mixing of its monomers: PTA and ethylene glycol (EG). Industries usually set the EG/PTA molar ratio to 1.2 - 1.3. At this ratio, the mixture of the two components results in the formation of a liquid paste displaying good processability for repolymerization. The relative amount of EG required to form this mixture, denoted here as EG/PTA ratio, is highly influenced by the particle size and morphology: it has been noted that in the case of oil-based PTA (particle size > 100 µm), only 0.9 eq. would be needed. On the other hand, using a poor-quality PTA (particle size < 1 µm), such as obtained from direct precipitation with H₂SO₄, the EG/PTA ratio can reach values higher than 2.0. The EG/PTA ratio can thus serve as a metric to characterize the quality/processability of the obtained PTA. The measured particle size is referring to the median value for longest distance in the particle, marked as x in Figure 1. The aspect ratio (AR), giving an information about the shape of the particle, is defined as y/x (AR = y/x). A value approaching 1 indicates a square or disc shape, while a value close to 0 indicates thin and long needles. Figure 1 shows the description of the aspect ratio of a PTA particle when AR = y/x, while x is the longest distance in the particle and y is the shortest distance in the particle.

### H₂SO₄ precipitation optimization

Samples of PTA obtained either by direct precipitation with a fast addition of concentrated H₂SO₄ or with several heat/cool cycles using acetic acid, were used as quality standards: the former as the starting point for H₂SO₄ optimization and the latter as the current benchmark. The experiments were directed to lower the EG/PTA ratio. A comparison of the crystals obtained with both methods is shown in Table 1.

**Table 1: Precipitation methods, the crystal size, and EG/PTA ratio determined for the resulting PTA.**

| **Acid** | **Method** | **Crystal size (µm)** | **EG/PTA** |
|---|---|---|---|
| AcOH | heat/cool cycles | 59.8 | 1.4 |
| H₂SO₄ | fast addition | < 5.0 | 2.6 |

The first goal of the optimization was to replace acetic acid with the cheaper sulfuric acid. In addition to its favourable price per litre, using a strong acid presents the advantage of necessitating fewer molar equivalents (rel. to M-TP) than the weak one.

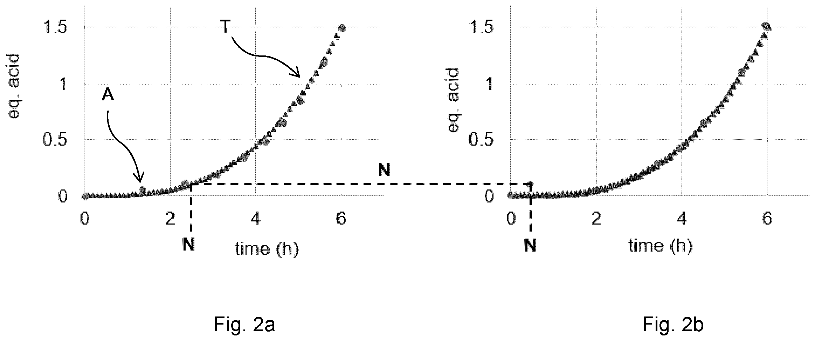

In order to avoid uncontrolled precipitation, leading to small size and aggregated particles with fast addition of H₂SO₄ (see Table 1), slow addition method, and in particular with a cubic addition profile, was tested (see Figures 2a and 2b).

In a first set of experiments, summarized in Table 2, the influence of sulfuric acid concentration and time on the PTA quality was tested.

**Table 2. Experimental details for experiments 1 to 3.**

| **Exp. n°** | **Initial pH** | **[Na-TP] (M)** | **[H₂SO₄] (M)** | **H₂SO₄ (eq.)** | **Time (h)** | **Particle size (µm)** | **AR** | **EG/PTA** |
|---|---|---|---|---|---|---|---|---|
| **1** | 8.6 | 0.28 | 0.36 | 1.5 | 6 | 30.8 | 0.47 | 1.7 |
| **2** | | | 0.71 | | | 48.6 | 0.46 | 1.5 |
| **3** | | | | | 4 | 30.6 | 0.47 | 1.8 |

Comparing the results shown in Table 2, entries 1 and 2, the concentration of H₂SO₄ was fixed to 0.71 M. This concentration resulted in the better product quality: comparable aspect ratio to acetic acid but larger particle size and lower EG/PTA ratio. Shortening the duration, however, initially seemed to lead to a lower quality (Table 2, entry 3, and Figure 2a).

It was then noticed that the initial of 0.10 - 0.15 eq. could be added faster in a fast addition, with a simpler linear rate, as they are only necessary to adjust the pH. After this point, the pH is low enough that the precipitated PTA cannot fully redissolve. This can be described as nucleation point. The following experiments were thus designed so that the addition profile would reach the nucleation point faster which resulted in maximizing the duration of the crystal growth period. A comparison of the two addition profiles is shown in Figure 2a and Figure 2b.

Figure 2a shows the addition profile without the initial fast acid addition and Figure 2b shows the addition profile with the initial fast acid addition within 15 minutes. The ideal cubic (black triangles, marked as T) and experimental profiles (grey discs, marked as A) are superimposed. The dashed black line indicates the approximate nucleation point and the corresponding time. In Figure 2b can be seen that an initial fast acid addition within 15 minutes leads to a faster reach of the nucleation point and to a faster reach of the beginning of crystal growth.

Using this multi-step addition profile, it was further investigated the effect of time on the PTA quality. The results are summarized in Table 3.

**Table 3. Experimental details for experiments 4 to 7.**

| **Exp. n°** | **Initial pH** | **[Na-TP] (M)** | **[H₂SO₄] (M)** | **H₂SO₄ eq.** | **Time (h)** | **Particle size (µm)** | **AR** | **EG/PTA** |
|---|---|---|---|---|---|---|---|---|
| **4** | 8.6 | 0.28 | 0.71 | 1.5 | 6 | 47.2 | 0.47 | 1.6 |
| **5** | | | | | 10 | 57.3 | 0.48 | 1.4 |
| **6** | 7.3 | | | | 15 | 56.8 | 0.47 | 1.4 |
| **7** | 7.6 | 0.29 | 0.71 | 1.0 | 4 | 31.3 | 0.60 | 1.4 |

These experiments showed that increasing the duration of the addition results in larger particles and improved EG/PTA ratio. The similar values were obtained for a total addition time of 10 and 15 hours (Table 3, entries 5 and 6 respectively). The size limit is reached after about 10 hours. Thus, the best results are obtained when the total addition time was between 4 to 10 hours. If the total addition time is less than 4 hours, the particle size is not large enough to fulfil the industrial standard. After 10 hours, the quality still remains good, but the additional time results in additional operation costs and is less economic. The quality parameter of the aspect ratio (AR) and of the EG/PTA ratio of all results fulfil the industrial standards.

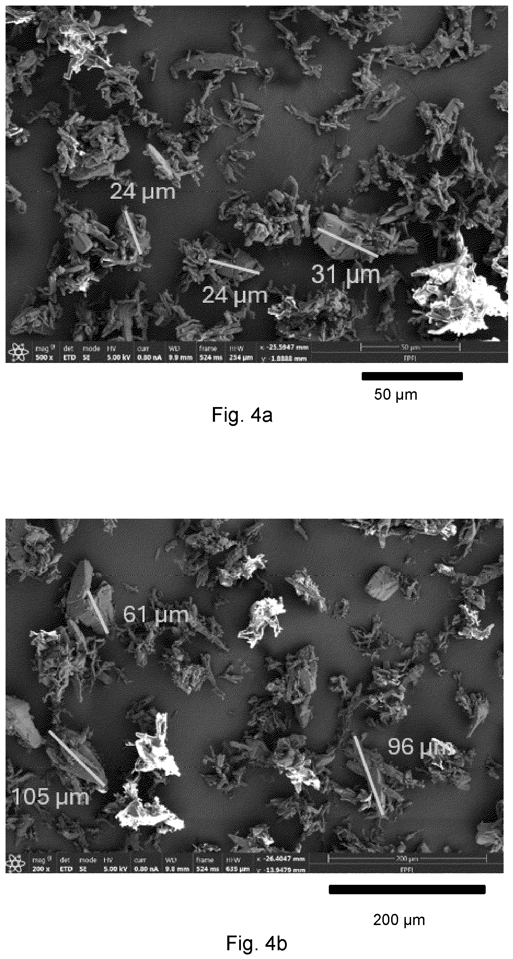

Switching from acetic to sulfuric acid and reducing the precipitation time from 6 to 4 h already represent important improvements in terms of operation costs. These different sets of conditions did, however, not allow to increase the PTA quality significantly. Thus, in a second step the precipitation is repeated and the previously in a first round obtained PTA were used as seed. The combination of both the slow addition approach and seeding allowed to significantly improve the quality of the produced PTA (see Figure 4b). The optimized method, detailed bellow, involves the production of good quality PTA crystals following the conditions shown in Table 3, entry 7.

### Experimental part

A disodium terephthalate (Na-TP) aqueous solution (34 mmol, 120 mL of a 0.29 M solution, 1.0 eq., pH 7.0) was heated at 85 °C while stirring. A diluted solution of H₂SO₄ (34 mmol, 48.2 mL of a 0.71 M aqueous solution, 1.0 eq.) was then added dropwise at 85 °C over a total addition time of 4 h (the addition profile is detailed below) under continuous agitation. The addition rate after the first fast addition step was adjusted to follow a cubic curve until the total addition time reached 4 hours and the amount of added acid reached 1 molar equivalent respectively. After completion, the suspension was cooled down to room temperature, using a water bath, and filtered. The solid was washed with deionised water until the conductivity of the filtrate reached a value below 50 µS/cm. The purified terephthalic acid (PTA) cake was collected and dried overnight in the oven at 100 °C. The experiment was repeated with a fresh Na_TP solution by introducing 10 wt.% (relative to the total weight the PTA in the Na-TP solution) of grinded PTA powder, also called PTA seeds, between the first fast addition step of H₂SO₄ and the second slow addition step of H₂SO₄. This allows to significantly increase the quality of the obtained PTA.

### H₂SO₄ addition profile

The multi-step sulfuric acid (H₂SO₄₎ addition of the invention is composed of several steps (see details in Table 4). In a first step, the H₂SO₄ solution is added in a fast linear addition with a fast rate up to 0.13 eq of H₂SO₄ during a first addition time of 15 minutes. At this point, described as nucleation (see Nucleation Point N in Figures 2a and 2b), the pH is low enough that the precipitated PTA cannot fully redissolve. If seeding is performed, the PTA seeds are added at this point which corresponds to step (c)(ia) of the invention, between the steps (c)(i) and (c)(ii), after step (c)(i) and at the beginning of (c)(ii). The addition rate is then significantly slowed down for the duration of a second step which corresponds to step (c)(ii) of the invention, and the addition rate is adapted to follow a cubic profile in the last part until a total addition time reaches 4 hours and the added acid reaches 1 molar equivalent of H₂SO₄ (steps 2 to 6 of Table 4). The addition profile is designed to favour the formation of PTA crystals with the desired characteristics (size and shape).

**Table 4. The different steps as programmed to follow the desired addition profile**

| **Step** | **Rate (mL/h)** | **Added volume (mL)** | **Step duration (h)** | **V(H₂SO₄)ₜₒₜ (mL)** | **Eq. H₂SO₄** | **Total time (h)** |
|---|---|---|---|---|---|---|
| **1** | 24 | 6.0 | 0.25 | 6.0 | 0.13 | 0.25 |
| **2** | 2 | 4.2 | 2.10 | 10.2 | 0.21 | 2.35 |
| **3** | 14 | 6.0 | 0.43 | 16.2 | 0.34 | 2.78 |
| **4** | 20 | 8.0 | 0.40 | 24.2 | 0.50 | 3.18 |
| **5** | 25 | 9.0 | 0.36 | 33.2 | 0.69 | 3.54 |
| **6** | 32 | 15.0 | 0.47 | 48.2 | 1 | 4.01 |

Figure 3 shows an H₂SO₄ addition profile. The multi-step addition profile of the invention is shown with the grey discs and lines, while the classical cubic profile is shown with the black triangles for comparison. Each point of the discs represents a different step of the profile, marked by a variation of the addition rate. The nucleation point, marked with N, is reached after 0.25 hours, when the crystals start to grow. The slow addition of sulfuric acid is started at the nucleation point N, also marked with C.ii, corresponding to the step (c)(ii) of the invention, starting slowly following the cubic curve.

Comparing previous methods using acetic acid (Table 5, entry 1), and the H₂SO₄ multi-step addition including the slow addition (Table 5, entry 2), similar EG/PTA values are obtained. This inventive method with the multi-step addition already shows improvements both on the acid consumption and the duration. With the additional seeding step (Table 5, entry 3) the crystal size significantly improves two times while conserving the aspect ratio. This also results in a drastic reduction of the EG/PTA value from 1.4 to 1.2. This value falls in the range of what is favourably used in the PET polymerization industry fulfilling the industrial standard. Thus, the combination of the multi-step H₂SO₄ addition including slow addition with the seeding step results in optimized crystalline PTA particles used for PET polymerization.

**Table 5: Precipitation methods and the related size, aspect ratio and EG/PTA values for the obtained PTA particles.**

| **Entry** | **Conditions** | **Median particle size (µm)** | **Aspect ratio (AR)** | **EGITPA** |
|---|---|---|---|---|
| **1** | AcOH | 59.8 | 0.40 | 1.4 |
| **2** | H₂SO₄, multi-step/slow | 31.3 | 0.60 | 1.4 |
| **3** | H₂SO₄, multi-step/slow (seeded) | 58.0 | 0.58 | 1.2 |

In Figures 4a and 4b, a scanning microscope image (SEM) of PTA particles are shown. In Figure 4a the crystals were obtained with the crystallization method without seeding, and in Figure 4b the crystals were obtained with the crystallization method of the invention combining the multi-step H₂SO₄ addition including slow addition with the seeding step. Without seeding, the crystal size is approx. 30 microns, which can be used as monomers for PET polymerisation. In Figure 4b, the particle size of the PTA crystals obtained with the method including the seeding step are between 30 and 100 microns or even larger and thus have an improved quality that is easier to process when later used for PET polymerisation. In Figure 4a the length of the black bar represents the distance of 50 µm, while in Figure 4b the length of the black bar represents the distance of 200 µm.

### Reference signs

- A: Dots/discs representing the steps of the addition method
- N: Nucleation point / point of seeding
- T: Triangles representing the ideal cubic curve
- C.ii: Beginning of step (c)(ii) of the slow addition
- C.ia: Step (c)(ia) and point of seeding with crystal seeds

## Claims

1. A crystallization method for purified terephthalic acid (PTA) to obtain crystalline PTA with an average crystal size of approx. 30-150 microns, the method comprising in the following order the steps of:
a. providing in a reactor a solution of 2-13 wt% of purified metalated salt of terephthalic acid (M-TP) in water at a pH of 7;
b. heating the M-TP solution to a crystallization temperature of 40-90°C and stirring the solution;
c. adding H₂SO₄ dropwise at the crystallization temperature over a total addition time of at least 4 hours under continuous agitation performing in the following order the steps of:
i. adding H₂SO₄ in the amount of 0.10-0.15 molar equivalent to adjust the pH of the M-TP solution to 4.7 - 5.5, which is called nucleation point, until the formation of a suspension of PTA to receive a first suspension of purified terephthalic acid (PTA);
ii. adding H₂SO₄ in the amount of 0.5-1.5 molar equivalent to the first suspension of PTA following a linear addition, a quadratic addition or a cubic addition with a predefined addition rate until the total addition time and 0.5-1.5 molar equivalent of H₂SO₄ is reached receiving a second suspension of PTA, wherein the predefined addition rate of the linear addition is defined by V(t) = Vf * (t / tf), the predefined addition rate of the quadratic addition is defined by V(t) = Vf * (t / tf)^2, and the predefined addition rate of the cubic addition is defined by V(t) = Vf * (t / tf)^3, where V(t) is the added acid volume at a given time, Vf is the total added acid volume, t is the time and tf is the total addition time;
d. cooling down the second suspension of PTA to room temperature;
e. filtering the cooled second suspension of PTA to collect crystalline PTA;
f. washing the filtered crystalline PTA with cold water; and
g. drying the washed crystalline PTA.

2. The method according to claim 1, wherein the first suspension of PTA after step (c)(i) is seeded with crystalline PTA in a step (c)(ia).

3. The method according to claim 2, wherein the crystalline PTA seeds have an average crystal size smaller than the average crystal size of the crystalline PTA of step (e).

4. The method according to one of claims 2 or 3, wherein the crystalline PTA seeds are added as a solid in the amount of 1-10 wt% of the total weight of PTA in the M-TP solution of step (a).

5. The method according to one of claims 2 to 4, wherein the crystalline PTA seeds are produced by a method of claim 1.

6. The method according to one of the preceding claims, wherein the crystallization temperature is between 80-90°C.

7. The method according to one of the preceding claims, wherein the method is performed under normal atmospheric pressure.

8. The method according to one of the preceding claims, wherein the metalated salt of terephthalic acid (M-TP) is either sodium therephthalate (Na-TP) or potassium therephthalate (K-TP).

9. The method according to one of the preceding claims, wherein the M-TP is obtained from depolymerization of waste PET via alkaline hydrolysis.

10. The method according to one of the preceding claims, wherein the M-TP is purified using graphite, activated carbon and molecular sieve to remove organic and inorganic contaminants.

11. The method according to one of the preceding claims, wherein the cold water of step (f) has a temperature of approx. 5 to 25°C.

12. The method according to one of the preceding claims, wherein the addition of H₂SO₄ in step (c)(ii) follows a cubic addition with a predefined addition rate defined by V(t) = Vf * (t / tf)^3.
